Europäisches Patentamt

European Patent Office

Office européen des brevets

(19)

(11) Publication number: **0 104 043**
A2

# EUROPEAN PATENT APPLICATION

(12)

(21) Application number: 83305367.1

(22) Date of filing: 14.09.83

(51) Int. Cl.³: **A 23 D 5/00**
**A 23 L 1/34, A 23 C 9/152**
**A 23 L 2/38, A 61 K 31/33**

(30) Priority: 20.09.82 JP 163732/82
15.02.83 JP 23460/83
19.08.83 JP 151348/83
19.08.83 JP 151349/83
19.08.83 JP 151350/83
19.08.83 JP 151351/83

(43) Date of publication of application:
28.03.84 Bulletin 84/13

(84) Designated Contracting States:
DE FR GB NL

(71) Applicant: AJINOMOTO CO., INC.
5-8, Kyobashi 1-chome, Chuo-ku
Tokyo 104(JP)

(72) Inventor: Kobayashi, Takaaki
No. 1197-14, Hirado-cho Totsuka-ku
Yokohama-shi Kanagawa-ken(JP)

(72) Inventor: Sekine, Seikichi
No. 6-10-19, Chiyoda
Sagamihara-shi Managawa-ken(JP)

(72) Inventor: Maekawa, Yoshio
No. 1152-2, Nakamaruko Nakahara-ku
Kawasaki-shi Kanagawa-ken(JP)

(72) Inventor: Suzuki, Hirohisa
No. 5-19-17, Tamanawa
Kamakura-shi Kanagawa-ken(JP)

(72) Inventor: Shimoda, Zenya
No. 1450, Shimotsuruma
Yamato-shi Kanagawa-ken(JP)

(72) Inventor: Ono, Ichiro
No. 1152-2, Nakamaruko Nakahara-ku
Kawasaki-shi Kanagawa-ken(JP)

(74) Representative: Bond, Bentley George et al,
HASELTINE LAKE & CO. Hazlitt House 28 Southampton
Buildings Chancery Lane
London, WC2A 1AT(GB)

(54) Foodstuffs and pharmaceuticals.

(57) A foodstuff or pharmaceutical which comprises incorporating α-monolinolein and/or β-monolinolein into a foodstuff or pharmaceutical in an amount of 1.0 wt% to 98 wt% calculated as monolinolein is disclosed. The thus obtained foodstuffs and pharmaceuticals possess an action of reducing blood cholesterol or an action of reducing increased blood cholesterol. Neither taste nor flavor is not damaged even when α-monolinolein and/or β-monolinolein are(is) added to foodstuffs.

EP 0 104 043 A2

—|—

## FOODSTUFFS AND PHARMACEUTICALS

The present invention relates to a process for preparing foodstuffs or pharmaceuticals containing a definite amount of α-monolinolein and/or β-monolinolein (hereafter collectively referred to as monolinolein). The foodstuffs and pharmaceuticals in accordance with the present invention possess an excellent action of lowing cholesterol level in blood or an excellent action of suppressing an increase of cholesterol level in blood.

The action of lowering cholesterol level in blood as used herein refers to an action of lowering cholesterol level in blood in the case where the cholesterol level is higher than normal cholesterol level. . Further the action of suppressing an increase of cholesterol level in blood as used herein refers to an action of suppressing cholesterol level in blood from constantly increasing even though foodstuffs containing large amounts of cholesterol are uptaken.

The term "foodstuff" as used in the present invention collectively refers to food edible to normal people including processed foodstuffs containing oils and fats (preferably foodstuffs containing at least 1.0 wt% of oils and fats therein) such as margarine, shortening, mayonnaise, dressings,

-2-

soup, chocolates, caramels, biscuits, cakes, donuts, breads, tofu or bean curd, rice cakes, etc., dairy products obtained by processing milk to be edible, such as cheese, butter, cream, condensed milk, powdered milk, fermented milk, ice cream, etc., beverages such as soybean milk, milk, juice, refreshing beverages, nutrient drink, carbonated drink, etc.

Based on findings in the past, it is a well known fact that polyunsaturated fatty acids (linoleic acid, etc.) contained in plant oils, plant sterols and the like possess an action of lowering cholesterol level or suppressing an increase of cholesterol level, in human and other animal bloods.

In recent years, reduction to practice has been attained as healthy foodstuffs in which substances of this type are supplemented and reinforced in foodstuffs or beverages for purposes of improving nutrition. Further in the field of pharmaceuticals, attention has been brought to physiological and pharmacological effects of these substances and in light of extensive investigations and results, pharmaceuticals have been developed and provided for use.

The present inventors previously filed Japanese Patent Application OPI Nos. 35517/82, 36935/82, 36936/82, 39736/82, 42631/82, etc. However, effective ingredients contained in compositions called for in these applications and edible oils and fats are contained in very small contents and it has not been clarified which compound would be effective. That is, these applications are directed to processes which comprises diluting a germ oil with an organic solvent, then cooling the dilution to -30 to -90°C, separating solid fractions, and fractionating from the solid fractions                 not eluted with a solvent having a solubility parameter of less than 7.5 but eluted with a solvent having a solubility parameter of not less than 7.5, stating that the action of lowing                         level
cholesterol or suppressing an increase of cholesterol level in blood is found in the thus collected fraction. However, it has not been clarified what would be an effective substance(s) in this fraction. Further, the prior art processes involve drawbacks that the yield of an effective substance(s) varies depending upon conditions for fractional procedures, etc.

Accordingly, an object of the present invention is to survey which is an effective substance in fraction mixtures in the aforesaid applications and to prepare foodstuffs or pharmaceuticals containing a specific amount of the thus surveyed effective substance and prepare foodstuffs or pharmaceuticals exhibiting an action of lowering                         level
cholesterol or an action of suppressing an increase of

0104043

cholesterol level in blood.

As a result of extensive investigations, paying attention to physiological effects of oil and fat components in the foregoing substances heretofore shown, the present inventors have found that monolinolein, a kind of monoglycerides, exhibits an action of lowering cholesterol level in blood in an amount of 1/100 that of substances conventionally employed and an action of suppressing an increase of cholesterol level in blood is equivalent thereto and, have accomplished the present invention.

That is, the present invention is directed to foodstuffs or pharmaceuticals which comprises incorporating α-monolinolein and/or β-monolinolein into foodstuffs or pharmaceuticals, for example, in an amount of 1.0 wt% to 98 wt% calculated as monolinolein.

The present invention is further directed to foodstuffs or pharmaceuticals which comprises incorporating α-monolinolein and/or β-monolinolein into foodstuffs or pharmaceuticals in an amount of, for example, 1.0 wt% to 98 wt% calculated as monolinolein and then, granulating, softening, capsulating or tableting the same.

The present invention is also directed to foodstuffs having good taste and flavor which comprises incorporating

-5-

α-monolinolein and/or β-monolinolein in an amount of ,for example, 1.0 wt%
to 98 wt% calculated as monolinolein into things edible to
normal people including processed foodstuffs containing oils
and fats (preferably foodstuffs containing at least 1.0 wt%
of oils and fats therein) such as margarine, shortening,
mayonnaise, dressings, soup, chocolates, caramels, biscuits,
cakes, donuts, breads, tofu or bean curd, rice cakes, etc.,
dairy products obtained by processing milk to be edible, such
as cheese, butter, cream, condensed milk, powdered milk,
fermented milk, ice cream, etc., beverages such as soybean
milk, milk, juice, refreshing beverages, nutrient drink,
carbonated drink, etc. in an amount of ,for example, 1.0 wt% to 98 wt%,
preferably 1.0 wt% to 30 wt%, calculated as monolinolein.

For preparing monolinolein employed in the present
invention, naturally occurring monoglycerides can be obtained
by isolation; further known synthesis methods including
direct esterification or ester exchange of glycerin using
catalysts such as alkalis, p-toluenesulfonic acid, etc. or
enzymes and, partial hydrolysis of triglycerides or diglycerides can also be utilized. These methods can also be used
in combination.

The monolinolein content contained in the compositions
and edible oils and fats called for in the previous applications (Japanese Patent Application OPI Nos. 35517/82, 36935/82, 36936/82, 39736/82 and 42631/82) by the present inventors is less than 1.0% and the effect is poorer than in the present invention. Further, the compositions are semi-solid substances containing 40 to 50% of plant sterols in addition to monolinolein. Accordingly, even though the compositions are incorporated into foodstuffs, foodstuffs having good taste and flavor are not obtained.

The term monolinolein as used herein refers to α-monolinolein and/or ß-monolinolein. In particular, ß-monolinolein has an excellent effect.

The thus obtained α-monolinolein and/or ß-monolinolein is incorporated in an amount of 1.0 wt% to 98 wt%, preferably 2 wt% to 5 wt%, based on the total amount. Materials in which α-monolinolein and/or ß-monolinolein is incorporated may be any materials as long as they are normally edible to human beings. That is, materials include animal and plant proteinous materials such as casein, soybean proteins, egg proteins, etc. Further, vitamin E, vitamin F, lecithin or the like may also be employed in combination. It is also possible to use monolinolein as a foodstuff, by incorporating it into oils and fats such as animal and plant oils, etc., edible materials such as carbohydrates, etc.

In the case of using in foodstuffs, it is particularly

—7—

preferred that monolinolein be incorporated into and dissolved in oils and fats. As oils and fats, there are
employed all of oils and fats including plant oils and fats
such as soybean oil, safflower oil, corn oil, cotton seed oil,
rape seed oil, rice oil, palm oil, olive oil, sunflower oil,
etc., animal oils and fats such as lard, tallow, cream, etc.,
a mixture thereof, or those processed by hydrogenation, ester
exchange, fractionation, etc. In the case of incorporating
monolinolein into beverages having a small content of oils
and fats, monolinolein may also be employed in combination
with other emulsifiers such as glycerine fatty acid esters,
sucrose-fatty acid esters, sorbitan fatty acid esters,
lecithin, etc..

The thus obtained formulation may be utilized as an
injection, a fluid, etc. In addition, the formulation may
also be employed after forming the same into medical
preparations such as soft agents, granules, capsules,
tablets, etc. In case that the formulation is converted into
medical preparations, binders, vehicles and other auxiliary
substances may be appropriately employed to mix them. Then,
the resulting mixture can be converted into medical preparations such as tablets, capsules, granules, pills, etc. by
means of a direct powder pressing method, a dry granule
pressing method, a wet granule pressing method, etc.

-9-

Examples of binders include starch, sugars, gelatin, gum arabic, methyl cellulose, etc. and examples of vehicles include calsium phosphate, calcium sulfate, sugars, starch etc. Monolinolein has no obstacle in accelerating a breakdown of medically preparaed forms and improving absorption in vivo due to emulsified dispersion.

These preparations can be utilized not only as single drugs but also as pharmaceuticals in combination with other drugs. Further, the medical preparations can also be employed as pharmaceuticals prepared for purpose of intravenous or rectal administration. In the case of using as a fluid for intravenous administration, at least three nutrients of fats, hydrated carbons and amino acids are incorporated and further vitamins, inorganic materials and the like are also formulated therein in a well balanced formulation from a dietetic viewpoint. That is, yolk phospholipids or soybean phospholipids are employed as emulsifiers in such a fluid; a part of or all of which can be replaced by monolinolein. Furthermore, monolinolein can be employed as emulsifiers for a sugar fluid, an amino acid fluid, a fat emulsion, etc. When monolinolein is employed in emulsifying fats in water under pressure, finely divided, stable oil-in-water type can be obtained. Further, monolinolein can also be incorporated, as a nutrient for rectal administration, into a nutrient food supplied through a tube, an elemental diet, etc., whereby the nutrient food is purified and absorbed in the intestinal tract.

In the present invention, monolinolein acts to lowering cholesterol level in blood or suppress an increase of cholesterol level in blood by uptake of monolinolein of the present invention as pharmaceuticals independently or in combination with other things, against hypercholesteremia due to various diseases such as arteriosclerosis, hyperlipemia, diabetes, liver disturbance, fatty liver, or, against increased cholesterol in blood which occurs even in normal people, when foodstuffs having a high cholesterol level are uptaken.

Monolinolein in accordance with the present invention has neither taste nor smell other than monoglyceride flavor which would be an obstacle in uptake and, is a highly stable substance.

Hereafter, the present invention will be explained in more detail with reference to the examples below.

-10-

## Example 1

1) Preparation of Monolinolein:

- Preparation from Corn Oil -

To 1 kg of purified corn oil, 10 kg of methyl ethyl ketone was added. After stirring the mixture, the mixture was cooled to -70°C for 3 hours using a dry ice-methanol coolant. Thereafter, solidified substances were removed by suction filtration and methyl ethyl ketone was removed from the remaining methyl ethyl ketone solution by distillation using an evaporator to obtain a red brown liquid. To the liquid, a 10-fold amount by weight of methyl ethyl ketone was added. Again by the same procedure, solidified substances at low temperatures were removed. Methyl ethyl ketone was removed by distillation to obtain 25 g of a red brown, highly viscous liquid.

22 g of this substance was developed, in sequence, with solvents shown in Table 1 through a glass column having an inner diameter of 70 mm in which 1500 g of activated "Wako Gel C-200" was packed. After recovering respective fractions, the solvents were removed by distillation to obtain four column-fractionated substances as shown in Table 2.

—||—

## Table 1.  Solvent for Column Development

| Fraction | Solvent for Development | Amount Used  (ml) |
|----------|------------------------|-------------------|
| I | 5% Diethyl Ether (in n-hexane) | 8000 |
| II | 20% Diethyl Ether (in n-hexane) | 4700 |
| III | 40% Diethyl Ether (in n-hexane) | 5000 |
| IV | Methanol | 4000 |

## Table 2

### Substances Fracationated by Column Chromatography

| Fracation | Yield (g) | Main Ingredients |
|-----------|-----------|-----------------|
| I | 3.1 | hydrocarbons, pigments, sterol esters |
| II | 5.9 | tocopherol, triglycerides |
| III | 5.4 | terpenes, free fatty acids |
| IV | 7.6 | monoglycerides, diglycerides, sterols |

The thus obtained Fraction IV was eluted with methanol : isooctane : water = 500 : 10 : 20 using a high performance liquid chromatography eqipped with Hitach Gel 3053 Column to isolate a fraction corresponding to a retention time of standard monolinolein. The thus isolated fraction was monoglycerides mainly composed of linoleic acid.  Isolation was repeated to obtain about 110 mg of monolinolein (purity 95%) from 7.6 g of Fraction IV.

2) Animal Test:

Male 28 days old rats of the Sprague-Dawley strain were fed a stock diet for 2 days. Then they were weighed to the nearest gram and divided into 2 groups of 10 rats on the basis of weight so that the average starting weight (81g) and weight range (78 to 84 g) were similar for each group. Animals were maintained individually in suspended cages in a room with regulated temperature (23 ± 1°C) and relative humidity (55 ± 10%). The light period was 12 hrs. (from 7:00AM to 7:00PM). Food and Water were available *ad libitum* and replenished daily throughout the 19-days experimental period. Before feeding the diets were stored at 7°C. The composition of experimental diet is shown in Table 3. Monolinolein was mixed with tallow and soybean oil (50 : 50) melted in a hot water bath. At the end of the 19-days feeding period, the rats were fasted for 16 hours. Then, blood was collected under ethereal anesthesia and serum was collected in a conventional manner. The total cholesterol in serum and β-lipoprotein in serum were measured using a kit, "Cholesterol B-Test Wako", manufactured by Wako Junyaku Kogyo Co., Ltd. and a kit, "β-Lipoprotein-Test Wako", manufactured by the same company, respectively. Performance, total cholesterol in serum and β-lipoprotein in serum are shown in Table 4.

-13-

## Table 3   Composition of Experimental Diet

### (Examples 1, 2 and 3)

| Ingredients | Percentage (%) |
|---|---|
| Sucrose | 59.29 or 58.29 |
| Casein | 20.0 |
| Cellulose | 4.0 |
| Mineral Mixture [1] | 4.0 |
| Vitamin Mixture [1] | 1.0 |
| Choline Chloride | 0.2 |
| Chocola A Drop [2] | |
| Chocola D Drop [2] | 0.2 |
| dl-α-Tocopherol Acetate | 0.01 |
| Cholesterol | 1.0 |
| Cholic Acid | 0.3 |
| Soybean Oil | 5 |
| Tallow | 5 |
| Monolinolein | 0 or 1.0 |

[1]:   manufactured by Oriental Yeast Industry Co, Ltd.,
         in which Harper  was formulated.

[2]:   manufactured by Eisai Co., Ltd.

Chocola A Drop 0.02%

diluted with water to
form a 0.2% solution

Chocola D Drop 0.01%

## Table 4

Effect of Monolinolein (prepared from corn oil) on Performance.

Total Cholesterol Levels and β-Lipoprotein Levels in Serum.

| | Initial Weight (g/rat) | Average Weight Gain (g/rat/ 19 days) | Average Feed Consumed (g/rat/ 19 days) | Feed Efficiency (%) | Total Chole- sterol in Serum (mg/dl) | β-Lipopro- protein in Serum (mg/dl) |
|---|---|---|---|---|---|---|
| Group 1: soybean oil (5%)+ tallow (5%)+ cholesterol (1.0%) | 81.6 ± 4.2 | 127.2 ±17.2 | 305.5 ± 24.2 | 41.6 ±4.3 | 198.8 ±44.4 | 419.2 ±12.8 |
| Group 2: Group 1 + monolinolein (1.0%) | 81.2 ±2.4 | 127.2 ±16.4 | 305.7 ±24.7 | 41.4 ±3.1 | 146.6* ±28.1 | 29.7.4* ±66.1 |

Significant Verification: * P <0.05

From this study , no influence was noted on average
weight gain, average feed consumed and feed efficiency
due to the addition of manolinolein. In total cholesterol
levels in serum and β-lipoprotein levels in serum, a significant
effect was noted by the addition of monolinolein.

-16-

## Example 2

1) Preparation of Monolinolein:

MONOLINOLEIN (Approx. 99%) manufactured by SIGMA CHEMICAL COMPANY in U.S.A. was used in this experiment.

2) Animal Test:

After feeding male 28 days old rats of the Sprague-Dawley strain with commercial diet for 5 days, the animals were divided into groups. Using the rats weighing 104.2 ± 4.1 g in average, each group being 10 rats, the rats were fed with each of test feeds for 17 days. Water was available *ad libitum* but food was consumed by paired feeding (feeding was conformed to the minimum feeding group).

The composition of expeimental diet shown in Table 3, only tallow was employed in an amount of 10% as an oil and fat, groups feeding feeds to which 1% of cholesterol and 0.3% of cholic acid were added and not added were supplemented and, monolinolein was replaced for sucrose having three levels of 0.5%, 1.0% and 2.0% in feeds and added to the feeds. Blood collection and meansurements of total cholesterol and $\beta$-lipoprotein in serum were carried out in a manner similar to Example 1.

## Table 5

Effect of Monolinolein (commercially available) on Performance.
Total Cholesterol Levels and β-Lipoprotein Levels in Serum.

| | Initial Weight (g/rat) | Average Weight Gain (g/rat/ 17 days) | Average Feed Consumed (g/rat/ 17 days) | Feed Efficiency (%) | Total Cholesterol in Serum (mg/dl) | β-Lipoprotein in Serum (mg/dl) |
|---|---|---|---|---|---|---|
| Group 1: Tallow (10%) | 104.5 ±4.9 | 39.5 ±6.9 | 248.7 ±1.7 | 36.0 ±2.8 | 72.8** ±17.1 | 132.6** ±34.6 |
| Group 2: Group 1 + Cholesterol (1%) | 104.5 ±4.4 | 93.1 ±9.5 | 245.6 ±5.6 | 37.9 ±3.5 | 433.1 ±108.1 | 1017.5 ±199.8 |
| Group 3: Group 2 + Monolinolein (0.5%) | 104.5 ±3.9 | 93.3 ±8.4 | 243.9 ±3.7 | 39.7 ±3.7 | 345.9 ±39.6 | 904.6 ±172.3 |
| Group 4: Group 2 + Monolinolein (1.0%) | 104.4 ±3.7 | 93.8 ±9.7 | 240.1 ±10.7 | 37.6 ±2.5 | 301.3 ±39.6 | 746.0 ±96.4 |

Table 5 (Continued)

Effect of Monolinolein (commercially available) on Performance, Total Cholesterol Levels and $\beta$-Lipoprotein Levels in Serum.

| | Initial Weight (g/rat) | Average Weight Gain (g/rat/ 17 days) | Average Feed Consumed (g/rat/ 17 days) | Feed Efficiency (%) | Total Cholesterol in Serum (mg/dl) | $\beta$-Lipoprotein in Serum (mg/dl) |
|---|---|---|---|---|---|---|
| Group 5: Group 2 + Monolinolein (2.0%) | 103.9 ±3.9 | 92.6 ±7.6 | 246.2 ±4.3 | 38.2 ±2.6 | 266.1* ±52.4 | 633.7** ±72.4 |

Significant Verification: to

    * : P<0.05

  ** : P <0.01

From this study , even though the levels of adding monolinolein were varied, the average weight gain and the feed efficiency were not affected at all; also in findings after autopsy, no particular abnormality other than liver hypertrophy due to cholesterol load was noted. Effect of monolinolein added was noted in an amount of 1.0% in the feeds, with respect to total cholesterol and $\beta$-lipoprotein in serum.

## Example 3

1)  Preparation of Monolinolein:

MONOLINOLEIN (Approx. 99%) manufactured by SIGMA CHEMICAL COMPANY in U.S.A. was used in this experiment as in Example 2.

2) Animal Test:

After feeding Male 28 days old rats of the Sprague-Dawley train with commercial diet for 5 days, the animals were divided into groups. Using the rats weighing 87.4 $\pm$ 4.4g in average, each group being 10 rats, the rats were fed with each of test diets for 17 days. Water was available *ad libitum* but food was consumed by paired feeding.

The composition of experiment diet was the same as in Example 2; monolinolein was added in an amount of 2.1%, $\beta$-lipoprotein and linoleic acid were added in an amount of

-20-

1%, respectively, singly or incombination, to the feed, in a manner similar to Example 1. Blood collection and measurements of total cholesterol and .

β- Lipoprotein in serum were carried out in a manner similar to Example 1.

In this experiment, the following were purchased and employed:

Linoleic acid: manufactured by Wako Junyaku Co, Ltd.

ß-sitosterol: manufactured by Wako Junyaku Co., Ltd.

## Table 6

Effect of Monolinolein (commercially available), β-Sitosterol and Linoleic Acid on Performance. Total Cholesterol Levels and β-Lipoprotein Levels in Serum.

| | Initial Weight (g/rat) | Average Weight Gain (g/rat/ 17 days) | Average Feed Consumed (g/rat/ 17 days) | Feed Efficiency (%) | Total Cholesterol in Serum (mg/dl) | β-Lipoprotein in Serum (mg/dl) |
|---|---|---|---|---|---|---|
| Group 1: Tallow (1%) | 86.0 ±4.4 | 82.1 ±7.4 | 225.5 ±1.2 | 36.4 ±3.2 | 98.3* ±14.1 | 192.7* ±41.3 |
| Group 2: Group 1 + cholesterol (1%) | 87.4 ±4.6 | 80.9 ±6.3 | 226.3 ±1.7 | 35.8 ±3.1 | 510.3 ±91.6 | 1372.7 ±304.1 |
| Group 3: Group 2 + monolinolein (2.1%) | 87.5 ±4.7 | 81.3 ±5.0 | 226.4 ±2.4 | 35.9 ±2.5 | 310.0 ** ±71.7 | 767.2** ±108.9 |
| Group 4: Group 2 + β-sitosterol (0.2%) | 87.5 ±4.1 | 80.5 ±2.6 | 227.1 ±1.1 | 35.5 ±1.1 | 349.7 * ±118.3 | 864.7* +283.7 |

Table 6 (Continued)

Effect of Monolinolein (commercially available), β-Sitosterol and Linoleic
Acid on Performance.  Total Cholesterol Levels and β-Lipoprotein Levels
in Serum.

|  | Initial Weight (g/rat) | Average Weight Gain (g/rat/ 17 days) | Average Feed Consumed (g/rat/ 17 days) | Feed Efficiency (%) | Total Cholesterol in Serum (mg/dl) | β-Lipoprotein in Serum (mg/dl) |
|---|---|---|---|---|---|---|
| Group 5: Group 2 + linoleic acid | 87.6 ±4.0 | 80.0 ±5.1 | 226.4 ±1.7 | 35.3 ±2.2 | 466.3 ±79.0 | 1312.2 ±169.7 |
| Group 6: Group 2 + monolinoleic acid (2.1%) + β-sitosterol (0.2%) | 87.5 ±3.9 | 82.9 ±5.6 | 227.9 ±1.1 | 36.4 ±2.5 | 388.3* ±110.0 | 391.7* ±297.2 |
| Group 7: Group 2 + mono- linolein (2.1%) + linolic acid (1%) | 87.4 ±3.7 | 83.5 ±6.2 | 226.8 ±1.0 | 36.8 ±2.5 | 377.9* ±81.6 | 939.5* ±233.0 |

0104043

## Table 6 (Continued)

Effect of Monolinolein (commercially available), β-Sitosterol and Linoleic Acid on Performance. Total Cholesterol Levels and β-Lipoprotein Levels in Serum.

| | Initial Weight (g/rat) | Average Weight Gain (g/rat/ 17 days) | Average Feed Consumed (g/rat/ 17 days) | Feed Efficiency (%) | Total Cholesterol in Serum (mg/dl) | β-Lipoprotein in Serum (mg/dl) |
|---|---|---|---|---|---|---|
| Group 8: Group 2 + monolinolein (2%) + β-sitosterol (0.2%) + linoleic acid (1.0%) | 87.5 ±3.6 | 81.8 ±4.6 | 227.9 ±1.1 | 35.9 ±2.0 | 287.1** ±73.3 | 669.3** ±171.0 |

Sibnivicant Verification: To Group 2:

    \* : $P < 0.05$

    \*\* : $P < 0.01$

-24-

From this study, even though monolinolein, ß-sitosterol and linoleic acid were added, the average weight gain and the feed efficiency were not affected at all; also in findings after autopsy, no particular abnormality other than liver hypertrophy due to cholesterol load was noted. With respect to total cholesterol and ß-lipoprotein in serum, a significant effect was noted with the addition of monolinolein alone even in an amount of 2.1%. It was noted that this effect was 3 times greater than that of ß-sitosterol and more than 200 times that of linoleic acid.

## Example 4

1) Preparation of Monolinolein:

MONOLINOLEIN (Approx. 99%) manufactured by SIGMA CHEMICAL COMPANY in U.S.A. was used in this experiment as in Example 2.

2) Animal Test:

After feeding Male 28 days old rats of the Sprague--Dawley strain with commercial diet for 5 dats, the animals were divided into groups. Using the rats weighing 85.0 ± 4.0g in average, each group being 10 rats,the rats were fed with each of test diets for 18 days. Food and water were available *ad libitum* . The composition of experimental diet is shown in Table 7. Blood collection and measurements of total cholesterol and β- lipoprotein in serum were carried out in a manner similar to Example 1.

-25-

## Table 7  Composition of Experimental Diet

(Example 4)

| Ingredients | Percentage (%) |
|---|---|
| Sucrose | 58.79 |
| Casein | 20.0 |
| Cellulose | 4.0 |
| Mineral Mixture *[1] | 4.0 |
| Vitamin Mixture *[1] | 1.0 |
| Choline Chloride | 0.2 |
| Chocola A Drop *[2] } Chocola D Drop *[2] | 0.2 |
| dl-α-Tocopherol Acetate | 0.01 |
| Corn Oil | 10 or 1 |
| Tallow | 0 or 10 |
| Monolinolein | 1.8 |

*1:  manufactured by Oriental Yeast Industry Co, Ltd.,
in which Harper was formulated.

*2:  manufactured by Eisai Co., Ltd.

Chocola A Drop 0.02%

} diluted with water to

form a 0.2% solution

Chocola D Drop 0.01%

## Table 8

Effect of Monolinolein (commercially available) on Performance, Total Cholesterol Levels and β-Lipoprotein Levels in Serum.

| | Initial Weight (g/rat) | Average Weight Gain (g/rat/ 18 days) | Average Feed Consumed (g/rat/ 18 days) | Feed Efficiency (%) | Total Cholesterol in Serum (mg/dl) | β-Lipoprotein in Serum (mg/dl) |
|---|---|---|---|---|---|---|
| Group 1:<br>Corn Oil (10%) | 85.1<br>±3.7 | 126.9<br>±14.9 | 306.4<br>±21.5 | 41.4<br>±3.7 | 71.2**<br>±11.3 | 179.5**<br>±34.1 |
| Group 2:<br>Tallow (10%) | 85.1<br>±4.2 | 118.0<br>±18.8 | 289.2<br>±28.9 | 40.8<br>±3.5 | 111.9<br>±17.6 | 313.6<br>±42.7 |
| Group 3:<br>Group 2 +<br>monolinolein (1.8%) | 85.4<br>±4.1 | 127.4<br>±17.8 | 301.6<br>±18.2 | 42.2<br>±3.5 | 80.9**<br>±9.2 | 203.3**<br>±39.8 |

Significant Verification: to Group 2,

**:    P<0.01

-27-

From this study, no difference among the groups was noted either in feeding results of the animals or in findings after autopsy. A significant effect was observed in the total cholesterol level and β-lipoprotein level in serum when 1.8% of monolinolein was added to the feed.

Example 5

1) Preparation of Monolinolein:

MONOLINOLEIN (Approx. 99%) manufactured by SIGMA CHEMICAL COMPANY in U.S.A. was purchased and used in this experiment as in Example 2.

-28-

2) Animal Test:

After feeding ⸢male 28 days old rats of the⸥ Sprague- Dawley strain with commercial diet for 5 days, the animals were divided into groups. Using the rats weighing 218.3 ± 17.7 g in average, each group being 10 rats, the rats were *ad libitum* fed for 17 days with each of feeds having the same composition as shown in Table 3 except that tallow alone was added in an amount of 10% as the fat and oil, to which no monolinolein was added and, cholesterol and cholic acid were added in the same amounts as shown in Table 3. At this point of time, the total cholesterol in blood was measured with a part of the rats and, monolinolein was injected to the remaining rats from the tail vein once a day for three days under the condition that the aforesaid feed obtained by loading cholesterol to tallow was consecutively fed to examine any influence on the cholesterol in blood.

The injections prepared by adding monolinolein in concentrations of 0.2% and 0.5%, respectively, were given to the rats, using as a control a physiological saline solution containing 2 mg/ml of Tween 80.

Table 9  Influence of Monolinolein on Total

Cholesterol Level in Blood When Intravenously Administered

| | Body Weight When Administered (g/rat) | Total Cholesterol Level in Serum (mg/dl) |
|---|---|---|
| Control Group:<br><br>Group 1:<br><br>  Physiological Saline<br>  Solution | $217.7 \pm 14.9$ | $434.4 \pm 47.4$ |
| Group 2:<br><br>  Group 1 +<br>  Monolinolein 4 (mg/kg)[2) | $215.8 \pm 16.7$ | $342.8 \pm 34.2$** |
| Group 3:<br><br>  Group 1 +<br>  Monolinolein 10 (mg/kg)[2) | $215.9 \pm 16.6$ | $301.3 \pm 39.6$** |

1)    2 mg/ml of Tween 80 was added.

2)    Dose per 1 kg of body weight

**    $P < 0.01$ (to Control Group)


From this results, it was clearly revealed that the administration of monolinolein by injection lowered the total cholesterol levels in serum.

-30-

## Example 6

1) Preparation of Capsules. Tablets and Granules:

(1) Preparation of Monolinolein:

MONOLINOLEIN (Approx. 99%) manufactured by SIGMA CHEMICAL COMPANY in U.S.A. was purchased and used in this experiment as in Example 2.

(2) Preparation of Capsules:

100 g of corn oil (manufactured by Ajinomoto Co., Ltd.) and 0.2 g of plant sterol (manufactured by Tama Seikagaku Co., Ltd.) were heated for 30 minutes to melt them. After cooling, 5 g of linoleic acid (manufacatured by Wako Junyaku Industry Co., Ltd.) and 0.5 g of vitamin E (manufactured by Nisshin Kagaku Co., Ltd.) were mixed with the resulting melt to obtain Mixture A. Further, 1 g of monolinolein was mixed with Mixture A to obtain Mixture B. These two mixtures were encapsulated using 65 g of gelatin to obtain 500 mg/grain of Capsule A (no monolinolein was added) and Capsule B (monolinolein was added), respectively.

(3) Preparation of Tablets:

To Mixtures A and B obtained in Preparation of Capsules, 500 g of corn starch (manufactured by Shionogi Pharmaceutical Co., Ltd.), 50 g of carboxymethyl cellulose (manufactured by Gotoku Yakuhin Co., Ltd.) and 40 g of hydroxypropyl cellulose having a low degree of substitution

-31-

(manufactured by Shin-Etsu Kagaku Co., Ltd.) were added to mix. Further 5 ml of water was added and the mixture was kneaded. The kneaded system was grained and ground and then dried at 50°C over 6 hours. After adding 2 g of magnesium stearate (manufactured by Junsei Kagaku Co., Ltd.) to the dried system and mixing them, the mixture was tableted to obtain Table A (no monolinolein was added) and Table B (monolinolein was added).

(4) Preparation of Granules:

To Mixtures A and B obtained in Preparation of Capsules, 35 g of corn starch, 65 g of carboxymethyl cellulose and 40 g of hydroxypropyl cellulose having a low degree of substitution were added to mix. Further 110 g of a 50% ethanol aqueous solution was added to the mixture to knead. After graining the thus kneaded system, it was blow-dried to remove ethanol. Then drying was performed at 30°C over 6 hours and sieved to obtain Granule A (no monolinolein was added) and Granule B (monolinolein was added), having a grain size of 1410 to 500 μm.

2) Animal Test:

After feeding male 28 days old rats of the Sprague-Dawley strain with commercial diet for 5 days, the animals were divided into groups. Using the rats weighing 93.8 ± 3.4 g in average, each group being 10 rats, the rats were fed with

−32−

each of test feeds for 16 days. Water was available *ad libitum* but food was consumed by paired feeding.

The composition of experimental diets was the same as in Table 3 except that tallow was used in an amount of 10% as the oil and fat; for the sample addition groups, cholesterol and cholic acid were added in amounts of 1% and 0.3%, respectively. The capsules, tablets and granules were mixed with the feed after cutting the capsules with a surgical knife to expose the content and, grinding the tablets and the granules into powders in a motar.

Blood collection and measurement of the total cholesterol in serum were carried out in a manner similar to Example 1.

## Table 10

Influence of Monolinolein Capsules, Tablets and Granules on Performance, Total Cholesterol Level in Serum.

| | Initial Weight (g/rat) | Average Weight Gain (g/rat/ 19 days) | Feed Efficiency (%) | Total Cholesterol in Serum (mg/dl) |
|---|---|---|---|---|
| Group 1:<br>Tallow (10%) | 93.8<br>±4.0 | 88.0<br>±4.0 | 39.5<br>±1.6 | 89.7 **<br>±13.6 |
| Group 2:<br>Group 1 +<br>Cholesterol (1%) | 93.6<br>±3.9 | 88.3<br>±4.5 | 39.8<br>±1.8 | 481.6<br>±114.1 |
| Group 3:<br>Group 2 +<br>Capsule A (5%) | 93.3<br>±4.1 | 86.3<br>±5.4 | 38.9<br>±2.1 | 334.9<br>±96.8 |
| Group 4:<br>Group 2 +<br>Capsule B (5%) | 93.7<br>±4.2 | 87.6<br>±4.9 | 39.1<br>±1.9 | 277.4 **<br>±38.7 |

Table 10 (Continued)

Influence of Monolinolein Capsules, Tablets and Granules
on Performance, Total Cholesterol Level in Serum.

| | Initial Weight (g/rat) | Average Weight Gain (g/rat/ 19 days) | Feed Efficiency (%) | Total Cholesterol in Serum (mg/dl) |
|---|---|---|---|---|
| Group 5: Group 2 + Tablet A (5%) | 92.9 ±2.6 | 88.3 ±6.0 | 39.7 ±2.4 | 356.1 ±86.9 |
| Group 6: Group 2 + Tablet B (5%) | 91.6 ±1.9 | 86.9 ±5.7 | 38.8 ±2.4 | 273.9** ±59.1 |
| Group 7: Group 2 + Granule A (5%) | 92.0 ±3.7 | 87.6 ±5.9 | 38.6 ±1.8 | 365.9 ±101.6 |
| Group 8: Group 2 + Granule B (5%) | 92.4 ±4.0 | 88.1 ±4.6 | 39.0 ±1.6 | 298.1* ±76.3 |

** :P<0.01 ⎫
 * :P<0.05 ⎭ Significant Different from Group 2

-35-

From this study, even though 5% of the capsules, tablets and granules prepared by adding ß-sitosterol and linoleic acid and further adding monolinolein to the foregoing two were added, the average weight gain and the feed efficiency were not affected at all; also in findings after autopsy, no particular abnormality other than liver hypertrophy due to cholesterol load was noted. An effect of the addition in each of the experimental diet on the total cholesterol in serum was remarkable with any of the feeds to which monolinolein was added.

## Example 7

After feeding male 28 days old rats of the Sprague-Dawley strain with commericial diet for 2 days, the animals were divided into 4 groups. Using the rats weighting 94.4 ± 3.52 g, one group being 10 rats, the rats were fed with each of test feeds for 18 days. Food and water were available *ad libitum* . The composition of experimetal diet was the same as that shown in Table 3. Monolinolein and the fourth component in Example 1, Table 3. Monolinolein and the fourth component in Example 1, Table 2 were mixed with tallow and soybean oil (50 : 50) melted in a hot bath. Edible oil and fat to which monolinolein was added showed good taste and flavor; however, taste and flovor of an oil and fat to which the fourth component was added was not good.

-36-

Blood collection and the measurements of the total cholesterol and ß-lipoprotein in serum were carried out in a manner similar to Example 1.

The results are shown in Table 11.

## Table 11

### Effect of Monolinolein (commercially available) on Performance, Total Cholesterol Level and β-Lipoprotein in Serum.

| | Initial Weight (g/rat) | Average Weight Gain (g/rat/ 18 days) | Average Feed Consumed (g/rat/ 18 days) | Feed Efficiency (%) | Total Cholesterol in Serum (mg/dl) | β-Lipoprotein in Serum (mg/dl) |
|---|---|---|---|---|---|---|
| Group 1: soybean oil (5%) + tallow (5%) + cholesterol (1.0%) | 94.4 ±3.5 | 126.9 ±14.9 | 306.4 ±21.5 | 41.5 ±3.7 | 246.2 ±35.0 | 610.3 ±70.0 |
| Group 2: Group 1 + Fourth component (2.0%) | 94.2 ±3.2 | 118.0 ±18.8 | 288.2 ±28.9 | 41.0 ±3.5 | 194.7* ±38.4 | 469.2* ±108.0 |
| Group 3: Group 1 + monolinolein (1.0%) | 94.1 ±4.8 | 119.6 ±11.4 | 280.7 ±24.1 | 42.7 ±4.5 | 160.1** ±15.4 | 389.0** ±50.1 |

Table 11 (Continued)

Effect of Monolinolein (commercially available) on Performance,

Total Cholesterol Level and β-Lipoprotein in Serum.

| | Initial Weight (g/rat) | Average Weight Gain (g/rat/ 18 days) | Average Feed Consumed (g/rat/ 18 days) | Feed Efficiency (%) | Total Cholesterol in Serum (mg/dl) | β-Lipoprotein in Serum (mg/dl) |
|---|---|---|---|---|---|---|
| Group 4: Group 1 + monolinolein (2.0%) | 94.1 ±4.8 | 122.1 ±12.3 | 283.9 ±29.2 | 43.1 ±2.6 | 154.3** ±22.6 | 387.5** ±67.8 |

Significant Verification:

*: P<0.05          **: P<0.01

-39-

## Example 8

After feeding male 28 days old rats of the Sprague-Dawley strain with commercial diet for 2 days, the animals were grouped. Using the rats weighing 92.3 ± 3.2 g, one group being 10 rats, the rats were fed with each of test feeds for 19 days. Food and water were available *ad libitum* . The composition of experimental diet was the same as that shown in Table 3.

To 54 g of yolk, 60 ml of vinegar and 6 g of sodium chloride were added and the mixture was agitated with a whipper to form a homogeneous aqueous phase. The aqueous phase was divided into three portions, to a first portion of which 100 g of safflower oil was added, to a second portion 100 g of safflower oil having dissolved therein the fourth component of Example 1, Table 2 in an amount of 2.0% and further to a third portion 100 g of safflower having dissolved therein 1.0% of monolinolein was added to form O/W type emulsions. Using the emulsions, three kinds of mayonnaise were prepared. Each of them was mixed with the feed in an amount of 10 wt%, which was provided for test.

Blood collection and the measurements of the total cholesterol and ß-lipoprotein in serum were carried out in a manner similar to Example 1.

## Table 12

Influence of Monolinolein (commercially available) on Performance, Total Cholesterol Level and β-Lipoprotein Level in Serum.

| | Initial Weight (g/rat) | Average Weight Gain (g/rat/ 19 days | Average Feed Consumed (g/rat/ 19 days | Feed Efficiency (%) | Total Cholesterol in Serum (mg/dl | β-Lippoprotein in Serum (mg/dl) | Functional Evaluation by Panel | |
|---|---|---|---|---|---|---|---|---|
| | | | | | | | Taste | Flavour |
| Group 1: Safflower Oil + Mayonnaise | 92.3 ±3.2 | 123.3 ±13.9 | 304.2 ±20.5 | 40.5 ±3.5 | 242.4 ±34.1 | 603.1 ±72.6 | 4.8 | 4.6 |
| Group 2: Group 1 + Fourth Component (2.0%) | 92.0 ±2.8 | 115.9 ±17.9 | 286.0 ±27.1 | 40.6 ±3.6 | 207.4 ±33.8 | 506.3 ±83.6 | 3.0 | 3.2 |
| Group 3: Group 1 + Monolinolein (10%) | 91.8 ±2.9 | 120.3 ±11.9 | 283.8 ±27.9 | 42.3 ±3.9 | 174.3* ±27.8 | 403.6* ±78.2 | 4.8 | 4.8 |

Significant Verification * P<0.05

–41–

## Example 9

After adding monolinolein to purified soybean oil and edible, hardened soybean oil (melting point, 34°C) having a composition shown in Table 13 and thoroughly mixing the resulting mixture, monostearin, lecithin, carotene and vitamin A were added to the mixture to dissolve them. To the resulting solution an aqueous solution of salt, fragments and sodium dehydroacetate was added while stirring to emulsify in W/O type. The resulting emulsion was quenched and kneaded to prepare margarin containing monolinolein therein.

The product has the quite the same appearance as ordinary margarine; taste, flavor, etc. were not inferior to ordinary margarine.

### Table 13

#### Raw materials of Monolinolein-Containing Margarine

| | |
|---|---|
| Purified Soybean Oil | 38 g |
| Edible, Hardened Soybean Oil | 42 |
| (m.p. 34°C) | 2.7 |
| Water | 16 |
| Salt | 1 |
| Monosterin (emulsifier) | 0.2 |
| Lecithin (emulsifier) | 0.1 |
| Carotene (coloring matter) | trace |
| Fragments | trace |
| Sodium Dehydroacetate | trace |
| Vitamin A | trace |

-42-

This margarin had the same suppressing effect on blood cholesterol level as that in the mayonnaise obtained in Example 8.

## Example 10

1) Preparation of Dressing:

To purified corn oil monolinolein was added to dissolve it. To the solution, vinegar, yolk and spices were added and a paste of corn starch, salt and water was further added thereto. After emulsifying the mixture, the emulsion was passed through a colloidal mill to prepare a dressing.

Proportions for formulation are shown in Table 14.

## Table 14

| | |
|---|---|
| Purified Corn Oil | 40 |
| Monolinolein | 2 |
| Vinegar | 5.2 |
| Yolk | 7 |
| Spices | 0.4 |
| Corn Starch | 2.7 |
| Salt | 1.1 |
| Water | 41.6 |

-43-

This dressing had good flavor and test, and the same suppressing effect on blood cholesterol level as that in the mayonnaise obtained in Example 8.


## Example 11

1) Preparation of Monolinolein-Containing Biscuit:

Raw materials shown in Table 5 were mixed to prepare a base for biscuit.

### Table 15

#### Raw Materials for Monolinolein-Containing Biscuit

| | |
|---|---|
| Wheat Flour | 46 |
| Shortening | 21 |
| Monolinolein | 1.5 |
| Sugar | 20 |
| Defatted Powdered Milk | 1.8 |
| Baking Powder | 0.2 |
| Sodium Bicarbonate | 0.1 |
| Salt | 0.6 |
| Water | 7.3 |
| Egg | 1.4 |
| Vanilla | 0.1 |

After molding, the mold was put in an oven followed by

-44-

baking at 220°C for 15 minutes.

This biscuit had the same suppressing effect on blood cholesterol level as that in the mayonnaise obtained in Example 8.

## Example 12

1) Preparation of Monolinolein-Containing Chocolate:

Raw materials shown in Table 16 were mixed and ground into fine grains. Thereafter, the grains were purified, tempered, casted and cooled to prepared chocolate.

The thus obtained chocolate had quite the same flavor, melting at the mount, taste, etc. as those of ordinary one.

### Table 16

| | |
|---|---|
| Bitter Chocolate | 20 |
| Cacao Butter | 17 |
| Defatted Powdered Milk | 18 |
| Monolinolein | 2 |
| Powdered Sugar | 42.5 |
| Lecithin | 0.5 |
| Fragments | trace |

This chocolate had the same suppressing effect on blood cholesterol level as that in the mayonnaise obtained in Example 8.

-45-

### Example 13

After feeding male 28 days old rats of the Sprague-Dawley strain with commercial diet for 2 days, the animals were grouped. Using the rats weighing 104.3 ± 4.1 g, one group being 10 rats, the rats were fed with each of test feeds for 18 days. Food and Water were available *ad libitum*. The composition of experimental diet was the same as that shown in Table 3.

Fats were separated from milk using a cream separator. At the working step out of steps of preparing butter comprising steps of neutrallization of cream, sterilization, cooling, holding, charning, removal of butter milk, washing of butter, salt-addition, working, casting and wrapping, 20 g of the fourth component of Example 1, Table 2 and 10 g of monolinolein were added, respectively, per 1 kg of butter and subsequent steps were followed in a conventional manner to obtain butter. Three kinds of butter together with commercially available were added to the feed in an amount of 10 g each, which were provided for test.

Blood collection and the measurements of the total cholesterol and ß-lipoprotein in serum were carried out in a manner similar to Example 1.

The functional evaluation was conducted by five expert pannelers using a 5 point evaluation. The results are shown in Table 17.

## Table 17

Influence of Monolinolein (prepared from corn oil) on
Performance, Total Cholesterol Level and β-Lipoprotein
Level in Serum.

| | Initial Weight (g/rat) | Average Weight Gain (g/rat/ 19 days | Average Feed Consumed (g/rat/ 19 days | Feed Efficiency (%) | Total Cholesterol in Serum (mg/dl | β-Lippoprotein in Serum (mg/dl) | Functional Evaluation by Panel | |
|---|---|---|---|---|---|---|---|---|
| | | | | | | | Taste | Flavour |
| Group 1: Butter | 104.5 ±4.4 | 93.1 ±9.5 | 245.6 ±5.6 | 37.8 ±4.0 | 433.1 ±108.6 | 1017.5 ±109.8 | 4.6 | 4.8 |
| Group 2: Group 1 + Fourth component (2.0%) | 104.5 ±3.9 | 93.3 ±8.4 | 243.9 ±8.4 | 38.1 ±3.8 | 345.9 ±57.1 | 904.6 ±172.3 | 3.2 | 3.0 |
| Group 3: Group 1 + Monolino- | 103.9 ±3.9 | 92.6 ±7.6 | 246.2 ±4.3 | 37.8 ±4.3 | 296.1* ±62.8 | 689.1* ±113.5 | 4.6 | 4.6 |

Significant Verification * : P<0.05

-47-

### Example 14

In a process for preparing cheese which comprises heat-treatment of a mixture comprising proteins, fats and other milky components separated from a mixture of milk, cream, defatted milk, butter milk and whey using a rennet, casting, pressing, salt-addition and ripening, 15 g of monolinolein was added to the mixture obtained after heat-treatment per 1 kg followed by subsequent steps in a conventional manner. Thus cheese was prepared.

This cheese had the same suppressing effect on blood cholesterol level as that in the butter obtained in Example 13.

### Example 15

After concentrating fresh, clean, defatted milk to a 2/3 volume that of the original volume, the defatted milk was heated at 90°C for 15 minutes. After cooling to 45°C, a starter for yogurt (having a ratio of Bulgariens and Str. thermophilus of 1:1) was added in an amount of 2% and the mixture was fermented at 45°C for 4 hours in a container which had been previously sterilized and cooled. After adding 1.2% of monolinolein thereto, the mixture was cooled at 5°C for 8 hours to obtain yogurt.

—48—

This yogurt had the same suppressing effect on blood cholesterol level as that in the butter obtained in Example 13.

## Example 16

Ice cream was prepared by adding sugar and fragments to cream, adding gelatin thereto as a stabilizer, further adding egg and fruits, adding monolinolein to the resulting mixture and then freezing the mixture while blowing air thereto.

This ice cream had the same suppressing effect on blood cholesterol level as that in the butter obtained in Example 13.

While the invention has been described in detail and with reference to specific embodiments thereof, it will be apparent to one skilled in the art that various changes and modifications can be made therein without departing from the spirit and scope thereof.

CLAIMS

1.    A foodstuff or pharmaceutical characterized in that it has incorporated therein α-monolinolein and/or β-monolinolein in an amount of 1.0 wt% to 98 wt% calculated as monolinolein.

2.    A foodstuff or pharmaceutical as claimed in claim 1, wherein said α-monolinolein or β-monolinolein is β-monolinolein.

3.    A foodstuff or pharmaceutical as claimed in claim 1 or 2, wherein said foodstuff or pharmaceutical having said α-monolinolein and/or β-monolinolein incorporated therein  has been granulated, softened, encapsulated or tableted.

4.    A pharmaceutical as claimed in any of claims 1 to 3, in the form of a solution or emulsion suitable for intravenous or rectal administration.

5.    A foodstuff as claimed in any of claims 1 to 3, wherein said foodstuff is an edible oil and fat.

6.    A foodstuff as claimed in any of claims 1 to 3, wherein said foodstuff is an oil and fat-containing processed foodstuff containing at least 1.0 wt% of oil and fat therein.

7.    A foodstuff as claimed in any of claims 1 to 3, wherein said foodstuff is an edible dairy product obtained by processing milk.

8.    A foodstuff as claimed in any of claims 1 to 3, wherein said foodstuff is a beverage.